# EUROPEAN PATENT APPLICATION

(11) **EP 3 753 552 A1**
(43) Date of publication of application: **23.12.2020**
(21) Application number: 20176069.1
(22) Date of filing: 03.12.2015
(51) Int. Cl.: A61K 9/19, A61K 9/08, A61K 47/02, A61K 35/34, C12N 5/077, C12N 15/113, A61P 9/00, A61P 43/00

(54) **PROCESSES FOR PRODUCING STABLE EXOSOME FORMULATIONS**

(30) Priority: 03.12.2014 US 201462086742 P
(62) Divisional of application: 15865745.2
(71) Applicant: Capricor, Inc., Beverly Hills, CA 90211 (US)
(72) Inventor: KREKE, Michelle, CA, 90211 (US); SMITH, Rachel, CA, 90211 (US); HANSCOME, Peter, PA, 18301 (US); PECK, Kiel, CA, 90211 (US); IBRAHIM, Ahmed, CA, 90211 (US)
(74) Representative: Lee, Nicholas John

(57) **Abstract**

The invention encompasses methods for generating stable exosome formulations and encompasses stable exosome formulations. The exosome formulations encompass stable liquid exosome formulations and stable lyophilized exosome formulations. In some embodiments, the exosome formulations can be generated by ultrafiltration and diafiltration. The exosome formulations can be suitable for administration to a human.

## Description

### BACKGROUND OF THE INVENTION

Exosomes are membrane-bound vesicles. Hong et al., PLoS ONE 9(8): e103310. doi:10.1371/journal.pone.0103310. They are found in biological fluids, such as urine, plasma, and ascites. Id. Exosomes are generated by inward budding of endosomal multivesicular bodies. Id. The cargo of exosomes includes proteins/glycoproteins expressed on the cell membrane as well as molecules and soluble factors present in the cytosol of parental cells. Id. Exosomes normally have diameters ranging from 40-100 nm. Zhang et al., Oncology Letters 8: 1701-1706, 2014. Exosomes contain special proteins, lipids, RNA and micro-RNAs. Id.

Exosomes produced from cardiosphere-derived cells enhance angiogenesis and promote cardiomyocyte survival and proliferation. Ibrahim et al., 2014 May 8;2(5):606-19, which is hereby incorporated by reference. Exosomes produced from cardiosphere-derived cells are enriched in miR-146a.

The leading cause of death in the US remains heart disease. Kochanek et al., Natl Vital Stat Rep. 2011;60:1-116. Adjusting for an aging population, the global incidence and mortality from ischemic heart disease is decreasing due current standard of care improvements in major adverse cardiac events (MACE). Moran et al., Circulation. 2014;129:1493-1501. However, the result is an increasing number of heart attack survivors and disability years due to nonfatal ischemic heart disease, which contributes greatly to the overall global economic burden of ischemic heart disease. Id. This suggests a need now to shift from MACE improvements over current standard of care to improvements in quality of life, fitness and vitality for the surviving patients with chronic angina and heart failure. *Id.*

Cardiosphere-derived cells (CDCs) are in clinical testing. CDCs administered after a myocardial infarction (MI) in two clinical trials (CADUCEUS and ALLSTAR) have been shown to be safe and effective in reducing scar size and increasing viable myocardium. Exosomes represent a next generation therapeutic platform for regenerative medicine. These nano-sized extracellular membranous vesicles are potent delivery vehicles for functional messenger RNA (mRNA), microRNA (miRNA) and DNA molecules and growing evidence suggests they can impart similar therapeutic benefits as their parent cells. CDC-derived exosomes have been shown to recapitulate the effects of CDCs now in numerous preclinical models. de Couto et al., Circulation. 2014;130; Ibrahim et al., Stem Cell Reports. 2014;2:606-619; Tseliou et al., Circulation. 2014;130. Research has shown that CDCs secrete exosomes containing particular miRNAs that stimulate cardiomyocyte proliferation, spur angiogenesis, and improve functional recovery in MI models. The totality of the preclinical data demonstrate that exosomes represent a required, secreted active pharmaceutical ingredient (API) for CDCs' primary mechanism of action (MOA) related to cardiac tissue repair.

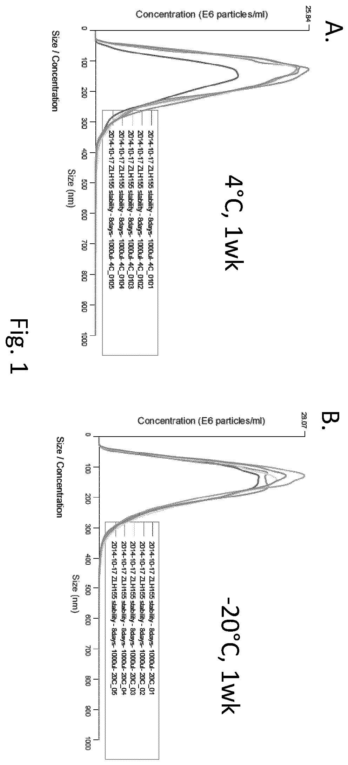

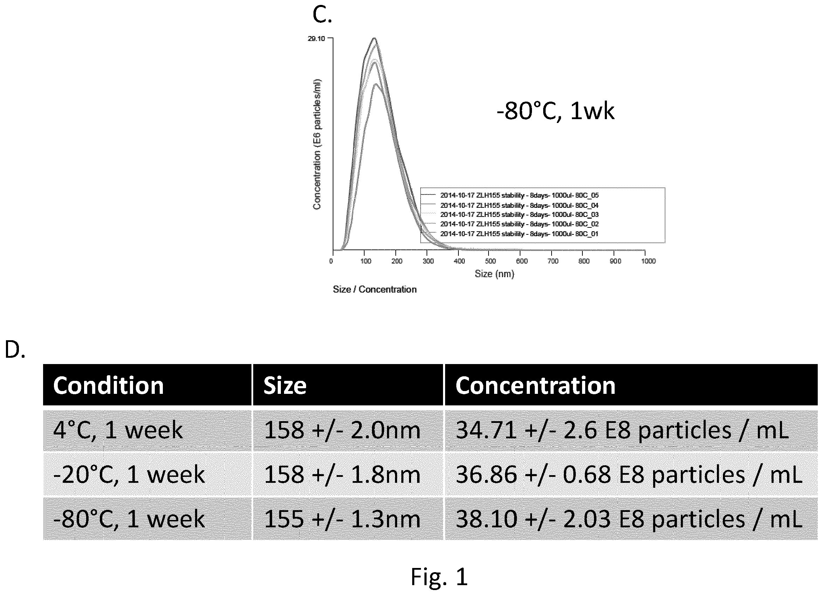

CDCs and their isolated exosomes hold great therapeutic potential to relieve this global burden of heart disease. CDCs in the Phase 1 CADUCEUS and ALLSTAR clinical trials have been shown to reduce scar size and increase myocardial tissue viability (see Figure 1). Malliaras et al., J Am Coll Cardiol. 2014;63:110-122; Makkar R et al., Lancet. 2012;379:895-904; Makkar et al., Journal of the American College of Cardiology. 2014;64.

CADUCEUS was the first clinical trial to observe increases in viable myocardium suggesting therapeutic regeneration. Makkar R et al., Lancet. 2012;379:895-904. The ongoing ALLSTAR Phase 2 trial with a 5 year sub-study will assess quality of life metrics and impact on hospitalization and mortality.

Nano-sized exosomes have major manufacturing and toxicology advantages over cells such as the ability to increase sterility assurance in the process using microbial retentive filters (e.g. ≤0.22 µm filters). Exosomes as non-living present potentially lower risks for adverse tumorigenic and immunogenic responses due to their very nature as non-living. Exosomes also certainly possess more flexibility in terms of stable drug storage temperature options compared to cells (e.g. room and cold temperatures vs. liquid nitrogen). The current research process for generating CDC-exosomes involves first seeding and growth of CDCs to confluence in fetal bovine serum (FBS)-containing conditions. For exosome production, the confluent layer of CDCs are washed and cultured under serum-free (free from FBS-exosomes), normoxic conditions for 15 days. Exosomes are then isolated from thawed, conditioned media (containing exosomes) using a precipitation method (intended for research use only), and formulated in base serum-free medium (a research grade reagent).

CDC exosomes are capable of improving cardiac function, stimulating angiogenesis and cardiomyocyte proliferation, and inhibiting cardiomyocyte apoptosis but not normal human dermal fibroblasts (NHDF) derived exosomes. When exosome secretion is inhibited using GW4869, the cardiac functional benefits of CDCs were diminished.

CDC exosome microRNA composition was characterized with a miRNA array. It was found that miR-210 and miR-146a were up-regulated in CDC exosomes in comparison to NHDF exosomes.

miR-210 is key player of the cellular response to hypoxia and capable of modulating cell survival and mitochondrial metabolism of both endothelial and cardiomyocytes. Hypoxia-inducible factor 1-alpha (HIF1α) directly binds to a hypoxia responsive element (HRE) on the proximal miR-210 promoter. The downstream targets of the HIF1α pathway is stromal cell-derived factor-1 (SDF-1) and vascular endothelial growth factor (VEGF). miR-210 is part of the key CDC exosome miRNA quantitative polymerase chain reaction (qPCR) panel to evaluate process parameters.

miR-146a is a pivotal immune regulatory molecule in various diseases and is induced upon the activation of toll-like receptor 4 (TLR4) in an nuclear factor kappa-light-chain-enhancer of activated B cells (NF-κB) -dependent signaling pathway which leads to the down regulation of interleukin 1 (IL-1) receptor-associated kinase 1 (IRAK1). The molecular targets of miR-146a involve the innate and adaptive immune responses and the CXCR4 pathway, which is a seven transmembrane G-protein coupled receptor of SDF-1. miR-146a is part of the key CDC exosome miRNA quantitative polymerase chain reaction (qPCR) panel to evaluate process parameters.

There is a need in the art for better exosome formulations, particularly for clinical use, and that are stable. The invention fulfills this need in the art.

### BRIEF SUMMARY OF THE INVENTION

The invention encompasses methods for generating stable exosome formulations and stable exosome formulations.

The invention encompasses a stable lyophilized exosome formulation suitable for administration to a human comprising at least 10⁶ exosomes, wherein the lyophilized exosomes maintain at least 90% of the levels of miR-210 RNA and miR-146A of the exosome formulation pre-lyophilization.

The invention encompasses a stable lyophilized exosome formulation suitable for administration to a human comprising at least 10⁶ exosomes, wherein the lyophilized exosomes maintain at least 90% of the level of in vitro biological activity of the exosome preparation pre-lyophilization.

The invention encompasses a stable lyophilized exosome formulation suitable for administration to a human comprising at least 10⁶ exosomes, wherein the lyophilized exosomes maintain at least 90% of the level of in vivo biological activity of the exosome preparation pre-lyophilization.

The invention encompasses a stable liquid cardiosphere-derived cell (CDC) exosome formulation suitable for administration to a human comprising at least 10⁶ CDC exosomes, wherein the exosome formulation maintains at least 75% of the levels of miR-210 RNA and miR-146A of the starting exosome formulation for 30 days at 4°C.

Preferably, the exosome formulation comprises at least 10⁸ exosomes.

Preferably, exosome formulation is generated from cardiosphere-derived cells (CDCs), most preferably by ultrafiltration and diafiltration.

### BRIEF DESCRIPTION OF THE DRAWINGS

FIG 1A-D depict CDC-derived exosome stability at various temperature conditions (4 to -80°C) after ultrafiltration (UFC) isolation as quantified by Brownian motion using the Nanosight nanoparticle tracking analysis.
FIG 2 depicts stable miR-146A and miR-210 expression in concentrated CDC-derived exosomes stored at -80°C for 7 to 90 days. Expression of miR-210 and miR-146A was lower when concentrated CDC-derived exosomes were stored at -20°C and 4°C for 30 to 90 days.
FIG 3 depicts similar upregulated miR-146A and miR-210 expression in formulated CDC-derived exosomes in PLASMALYTE A, RINGERS and PLASMALYTE A + Dextrose infusions solutions or BSS Plus flush solution compared to ultrafiltration (UFC) control.
FIG 4 depicts stable miR-146A and miR-210 expression in formulated CDC-derived exosomes in PLASMALYTE A, RINGERS and PLASMALYTE A + Dextrose and stored for -30 days at 4°C, -20°C and -80°C compared to UFC controls.
FIG 5 and 6 depict bioactivity of formulated CDC-derived exosomes in PLASMALYTE A (Plasma), RINGERS (Ring) and PLASMALYTE A + Dextrose (P+D) compared to UFC controls. Bioactivity indicated by macrophage phenotype change to a cardioprotective phenotype demonstrated by Arg 1 and Nos 2 upregulation. Dotted line indicates baseline expression (1) of the primary macrophages.
FIG 7A-C depicts the bioactivity of the lyophilized (freeze dried) CDC-derived exosomes as demonstrated by A) the macrophage phenotype change to a cardioprotective phenotype demonstrated by Arg 1 and Nos 2 upregulation and B) reduced scar mass in rat AMI reperfusion model. C) Expression of miR-146A and miR-210 was similar for ultrafiltration by centrifugation (UFC) and UFC lyophilized exosomes.
FIG 8 depicts upregulated miR-146A and miR-210 expression with CDC-derived exosomes before (Pre-Lyo) and post (Post-Lyo) lyophilization.
FIG 9 depicts pH variations with and without diafiltration. **Filtered CM:** The starting material, after collection but prior to concentration by ultra-filtration. **UFC:** Concentrated exosome product, no diafiltration. Representative of the starting pH prior to diafiltration. **PlasmaLyte:** Yielded consistent results in 6.9-7.2 range. **PlasmaLyte + Dextrose:** Similar results to those seen when using PlasmaLyte alone. **Ringers:** Comparatively large shift in pH compared to other buffers. Potential for very wide buffering capacity if necessary. **BSS+:** Large upward shift in pH.

### DETAILED DESCRIPTION OF THE INVENTION

The effect of storing exosomes at various concentrations was examined. (Figure 1.) In this experiment, the exosomes prepared by ultrafiltration were stored at 4°C, -20°C, and -80°C for 7 days. The total numbers of exosomes did not decrease substantially at various temperatures over these timeperiods

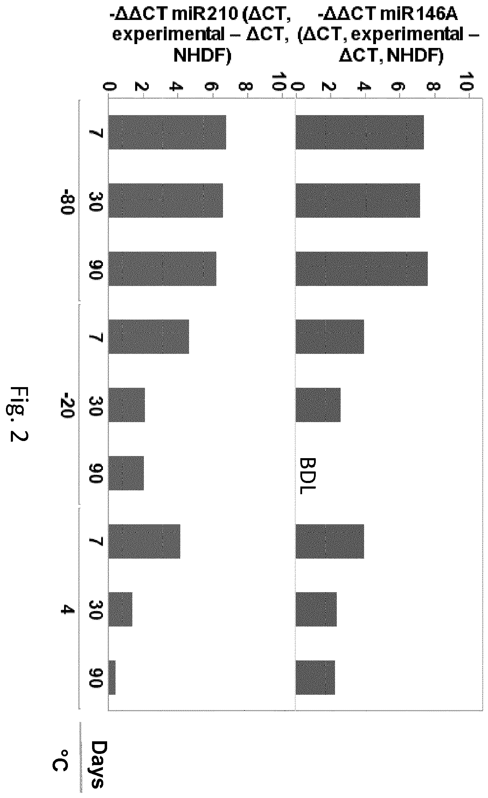

Bulk concentrated CDC-derived exosomes were prepared by ultrafiltration. When stored at -80°C for 7 to 90 days, concentrated CDC-derived exosomes showed stable miR-146A and miR-210 expression. (Figure 2.) Expression of miR-210 and miR-146A was lower when concentrated CDC-derived exosomes were stored at -20°C and 4°C for 30 to 90 days. At -20°C and 4°C, the exosomes contained less than 50% of miR-146A and miR-210 levels at 30 days.

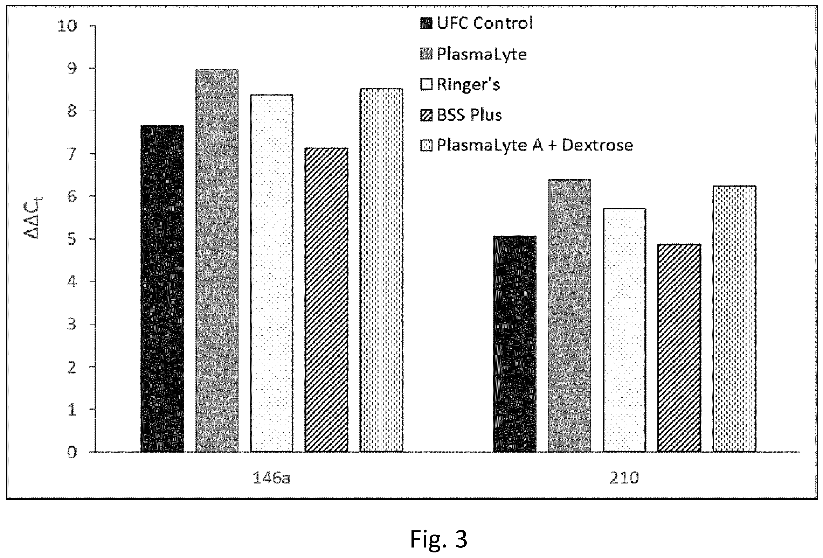

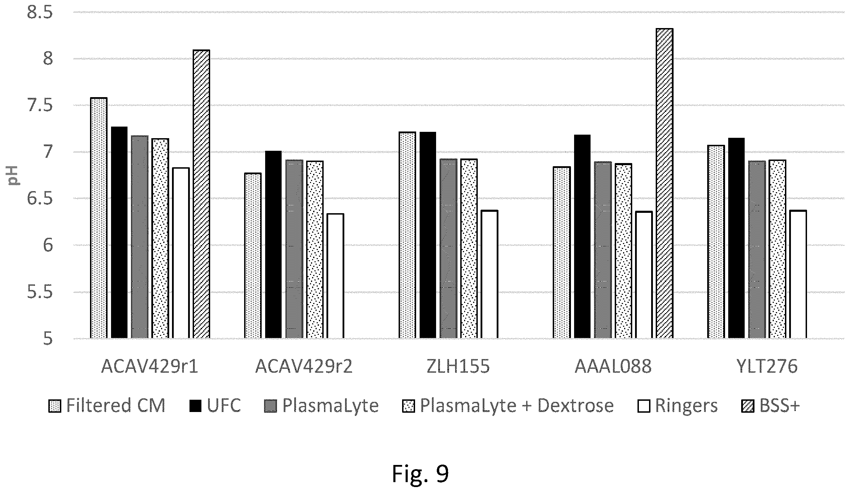

To try to improve exosome stability at -20°C and 4°C, CDC-derived exosomes were formulated by difiltration in PLASMALYTE A, RINGERS and PLASMALYTE A + Dextrose infusions solutions or BSS Plus flush solution and compared to an ultrafiltration (UFC) control. (Figure 3.) All of the formulations showed similar levels of miR-146A and miR-210 RNAs. However, relatively large pH changes were seen with Ringer's and BSS. (Figure 9.)

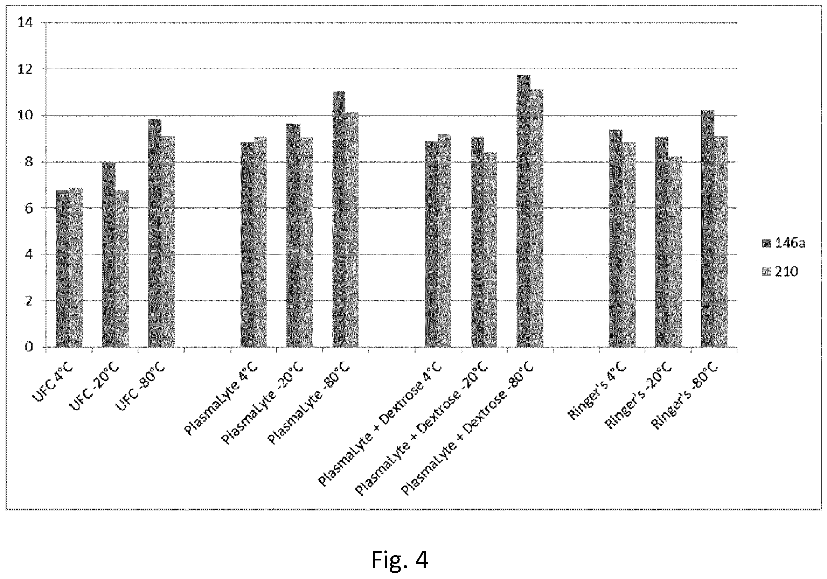

To compare long-term stability of various formulations, CDC-derived exosomes were formulated in PLASMALYTE A, RINGERS and PLASMALYTE A + Dextrose and then stored for -30 days at 4°C, -20°C and -80°C. UFC controls showed a reduction of about 20-30% of miR-146A and miR-210 levels at 4°C and -20°C, compared to storage at -80°C. (Figure 4.) All of the other formulation showed lower reductions, indicating that the stability of these RNAs was improved compared to the controls.

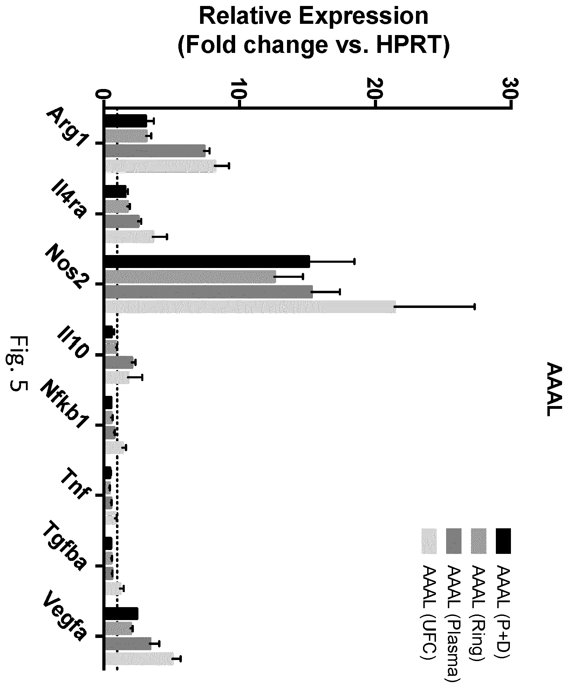

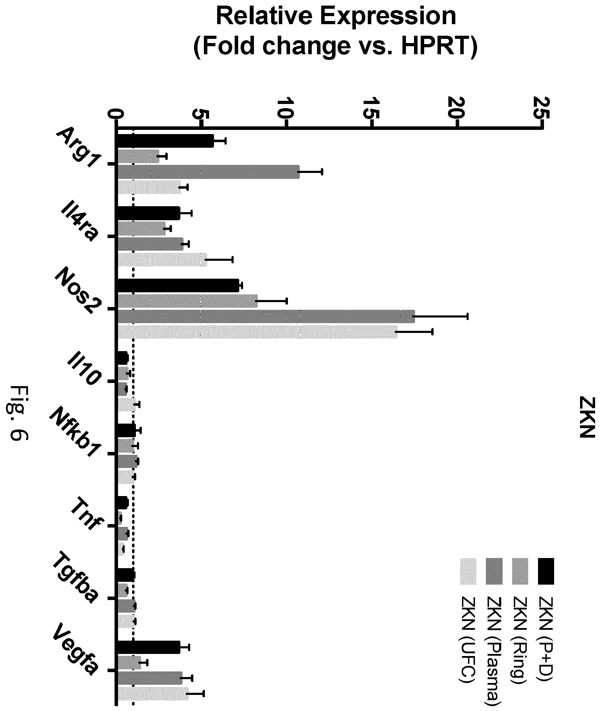

CDC-derived exosomes formulated in PLASMALYTE A (Plasma), RINGERS (Ring) and PLASMALYTE A + Dextrose (P+D) were compared to UFC controls in an in vitro bioactivity model. (Figures 5 & 6.) Although all preparations showed bioactivity, PLASMALYTE A showed the best bioactivity.

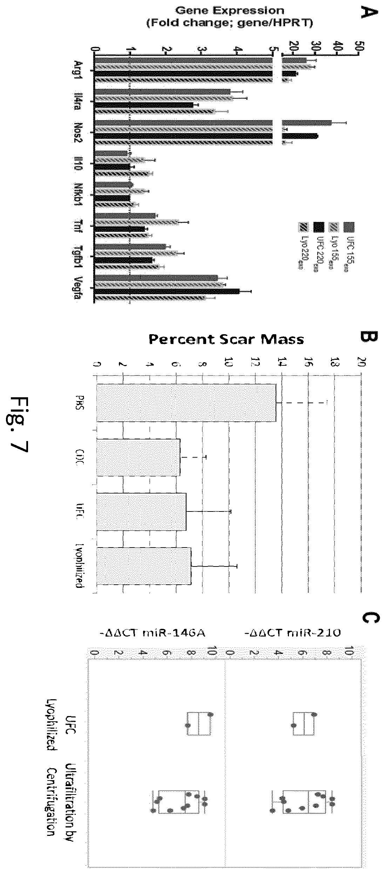

To determine whether exosomes could be lyophilized and retain their activity, initial experiments were performed to assess this approach. Using a speedvac caused great instability of the exosomes with loss of exosome bioactivity. Bulk UFC concentrated CDC-derived exosomes at least 10⁶ were lyophilized, while low temperatures were maintained during the conditions of sublimation, using the lyophilization protocol in Example 5, stored for at least 30 days at -80°C, and rehydrated in water. They were then tested in an in vitro macrophage bioactivity assay. (Figure 7A.) The lyophilized exosomes showed excellent bioactivity as demonstrated by the macrophage phenotype change to a cardioprotective phenotype, which was similar to non-lyophilized exosomes. Thus, the lyophilization and rehydration of the exosomes did not cause any significant reduction in *in vitro* bioactivity.

The lyophilized exosomes were also assessed for bioactivity in a rat AMI reperfusion model. (Figure 7B.) Lyophilized CDC-derived exosomes showed excellent bioactivity as demonstrated by reduced scar mass. Thus, the lyophilization and rehydration of the exosomes did not cause any significant reduction in *in vivo* bioactivity.

Expression of miR-146A and miR-210 was also assessed by PCR and similar levels were observed for ultrafiltration by centrifugation (UFC) and UFC lyophilized exosomes. (Figure 7C.) Thus, unexpectedly, exosomes can be lyophilized and rehydrated without causing significant losses of miRNAs and without causing losses in bioactivity, using both *in vitro* and *in vivo* assays.

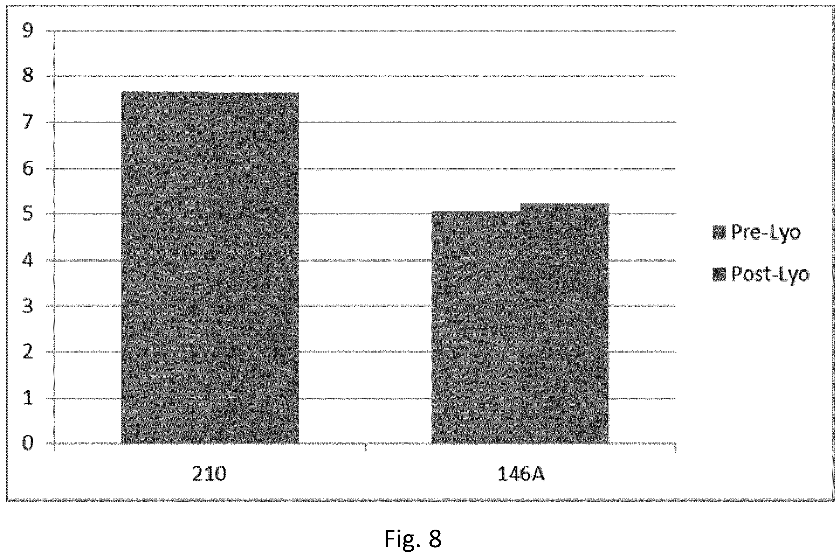

Similar levels of upregulated miR-146A and miR-210 expression with CDC-derived exosomes before (Pre-Lyo) and post (Post-Lyo) lyophilization and stored for at least 30 days at -80°C was observed. (Figure 8.) Thus, lyophilized exosomes can be stored for extended periods, while maintaining miRNA levels.

The invention allows for the generation of stable exosome formulations. The invention encompasses these exosome formulations and methods for producing them.

### Methods for generating exosomes

The invention encompasses methods for producing exosomes comprising culturing cells. The invention encompasses methods for generating exosomes comprising culturing cells in less than 20% oxygen for at least 2 days and harvesting exosomes from the cells.

Preferably, the cell culture comprises at least 10⁶, 10⁷, 10⁸, 10⁹, 10¹⁰, 10¹¹, or 10¹² cells.

Preferably, the cells are primary cells. The primary cells can be at least at passage number 2, 3, 4, 5, 6, 7, 8, 9, or 10. Immortalized cells are also encompassed by the invention.

Preferably, the cells are human cells. A particularly preferred cell type is cardiosphere-derived cells (CDCs). Other preferred cell types are cardiac tissue derived stem cells, adipose tissue derived stem cells, neural tissue derived stem cells and other tissue derived stem cells.

In one embodiment, the cells can be grown under routine culture conditions, for example, 20% O₂ at 37°C in IMDM with 20% fetal bovine serum (FBS) and pen/strep by seeding 10⁶ cells per T175 flask.

In various embodiments, the oxygen concentration is 1-2% 2-3%, 4-5%, 5-6%, 6-7%, 7-8%, 8-9%, 9-10%, 10-11%, 11-12%, 12-13%, 13-14%, 14-15%, 15-16%, 17-18%, or 18-19%. Preferably, the oxygen concentration is 2-8%, 3-7% oxygen, 4-6% oxygen, or 4.5-5.5% oxygen.

The cells can be cultured for at least 2, 3, 4, 5, 6, 7, 8, 9, 10, 11, 12, 13, 14, 15, 16, 17, 18, 19, or 20 days. Preferably, the cells are cultured for 5-15 days, 5-10 days, or 10-15 days.

Preferably, the cell culture comprises an insulin supplement and/or chemically defined lipid and cholesterol lipid concentrates.

### Exosome preparations

The invention encompasses exosome preparations generated from the cell cultures of the invention. In various embodiments, the exosome preparation contains exosomes of 50 nm to 250 nm in diameter. Preferably, at least 25%, 50%, 65%, 75%, 80%, 85%, 90%, or 95% of the exosomes are at least 50nm, 60 nm, 70 nm, 80 nm, 90 nm, 100 nm, 110 nm, 120 nm, 130 nm, 140 nm, 150 nm, or 160nm. Preferably, at least 25%, 50%, 65%, 75%, 80%, 85%, 90%, or 95% of the exosomes are less than 250nm, 240 nm, 230 nm, 220 nm, 210 nm, 200 nm, 190 nm, 180 nm, 170 nm, 160 nm, 150 nm, or 140 nm in diameter. Thus, the invention includes exosome preparations wherein at least 25%, 50%, 65%, 75%, 80%, 85%, 90%, or 95% of the exosomes are between 50 nm to 250 nm in diameter, 60 nm to 250 nm in diameter, 60 nm to 240 nm in diameter, 50 nm to 240 nm in diameter, etc.

In some embodiments, the exosome preparation contains at least 10⁵, 5x10⁵, 10⁶, 5x10⁶, 10⁷, 5x10⁷, 10⁸, 5x10⁸, 10⁹, 5x10⁹, 10¹⁰, 5x10¹⁰, 10¹¹, 5x10¹¹, or 10¹², or 5x10¹² exosomes. In some embodiments, the exosome preparation contains between 10⁵, 10⁶, 10⁷, 10⁸, 10⁹, 10¹⁰, or 10¹¹ to 10⁶, 10⁷, 10⁸, 10⁹, 10¹⁰, 10¹¹, or 10¹², etc. exosomes.

In some embodiments, the exosomes preparation includes one or more exosomes containing microRNAs. In various embodiments, these microRNAs can include miR-146A and/or miR-210. In some embodiments, the exosome preparation includes exosomes enriched in miR-210.

In various embodiments, the oxygen concentration in the culture of cells that generate the exosome is 1-2%, 2-3%, 4-5%, 5-6%, 6-7%, 7-8%, 8-9%, 9-10%, 10-11%, 11-12%, 12-13%, 13-14%, 14-15%, 15-16%, 17-18%, or 18-19%. Preferably, the oxygen concentration is 2-8%, 3-7% oxygen, 4-6% oxygen, 4.5-5.5% oxygen. Exosomes generated from cells cultured in a lower oxygen concentration (e.g., 2-8% oxygen) differ in their RNA and protein constituents from exosomes generated from cells cultured in 20% oxygen.

In one embodiment, the protein content of the exosomes generated from cells cultured in 2-8% oxygen is higher than that of exosomes generated from cells cultured in 20% oxygen. In one embodiment, the total RNA content of the exosomes generated from cells cultured in 2-8% oxygen is higher than that of exosomes generated from cells cultured in 20% oxygen. In one embodiment, the miR-146A RNA content of the exosomes generated from cells cultured in 2-8% oxygen is higher than that of exosomes generated from cells cultured in 20% oxygen. In one embodiment, the miR-210 RNA content of the exosomes generated from cells cultured in 2-8% oxygen is higher than that of exosomes generated from cells cultured in 20% oxygen.

### Stable liquid CDC exosome formulations

In some embodiments, the invention encompasses stable liquid exosome formulations. Within the context of the "stable liquid CDC exosome formulations" of this invention, the term "liquid" refers to the state of the formulation at 20°C. Thus, a stable liquid CDC exosome formulation can be a solid when it is frozen at -20°C. The phrase, "suitable for administration to a human" means that the formulation has been prepared in a pharmaceutically acceptable composition under conditions to maintain sterility of the formulation.

The stable liquid CDC exosome formulation can contain at least 10⁵, 5x10⁵, 10⁶, 5x10⁶, 10⁷, 5x10⁷, 10⁸, 5x10⁸, 10⁹, 5x10⁹, 10¹⁰, 5x10¹⁰, 10¹¹, 5x10¹¹, or 10¹², or 5x10¹² CDC exosomes. In some embodiments, the stable liquid CDC exosome formulation contains between 10⁵, 10⁶, 10⁷, 10⁸, 10⁹, 10¹⁰, or 10¹¹ to 10⁶, 10⁷, 10⁸, 10⁹, 10¹⁰, 10¹¹, or 10¹², etc. CDC exosomes. Preferably, the stable liquid CDC exosome formulation comprises at least 10⁶ or 10⁸ exosomes.

In some embodiments, the levels of miR-210 RNA and miR-146A in the stable liquid exosome formulation are maintained at a level of at least 10%, 20%, 30%, 40%, 50%, 60%, 70%, 80%, 90%, 95%, 99%, etc. of the starting level for at least 7, 14, 21, 23, 30, 60, 90, etc. days. Whether the levels of miR-210 RNA and miR-146A in the stable liquid exosome formulation are maintained at a level of at least 10%, 20%, etc. of the starting level for at least 7, 14, etc. days can be determined using the assays set forth in the Examples, and similar assays.

In some embodiments, the levels of miR-210 RNA and miR-146A are maintained at a level of at least 10%, 20%, etc. of the starting level for at least 7, 14, etc. days at 20°C, 10°C, 4°C, 0°C, -10°C, -20°C, -40°C, -80°C, etc. Preferably, the levels of miR-210 RNA and miR-146A are maintained at a level of at least 30% of the starting level for at least 30 days at 4°C, -20°C, or -80°C.

In some embodiments, the stable liquid exosome formulation comprises less than 1.0%, 0.1%, .01%%, 0.001%, 0.0001%, etc. polyethylene glycol (w/v). Preferably, stable liquid exosome formulation comprises less than .01% polyethylene glycol.

Preferably, the stable liquid exosome formulation is generated by ultrafiltration. Most preferably, the stable liquid exosome formulation is generated by ultrafiltration from cardiosphere-derived cells (CDCs) by ultrafiltration.

Preferably, the stable liquid exosome formulation comprises a solution containing at a level (per 500ml) of 2200 - 3000 mg of sodium chloride, 160 - 200 mg potassium chloride, approximately 150 mg of magnesium chloride hexahydrate, 70 mEq sodium, approximately 1800 mg sodium acetate trihydrate and approximately 2500 sodium gluconate.

Preferably, the stable liquid exosome formulation maintains at least 10%, 20%, 30%, 40%, 50%, 60%, 70%, 80%, 90%, 95%, 99%, etc. of the level of *in vitro* biological activity of the starting exosome formulation for at least 7, 14, 21, 23, 30, 60, 90, etc. days. The levels of *in vitro* biological activity can be determined using the macrophage assay set forth in the Examples using the techniques of de Couto et al., J Clin Invest. 2015 Aug 3;125(8):3147-62, which is hereby incorporated by reference.

Preferably, the stable liquid exosome formulation maintains at least 10%, 20%, 30%, 40%, 50%, 60%, 70%, 80%, 90%, 95%, 99%, etc. of the level *of in vivo* biological activity of the starting exosome formulation for at least 7, 14, 21, 23, 30, 60, 90, etc. days. The levels of *in vivo* biological activity can be determined using the rat AMI reperfusion model assay set forth in the Examples using the techniques of de Couto et al., J Clin Invest. 2015 Aug 3;125(8):3147-62, which is hereby incorporated by reference.

### Stable lyophilized exosome formulations

In some embodiments, the invention encompasses stable lyophilized exosome formulations. Within the context of the "stable lyophilized exosome formulations" of this invention, the term "lyophilized" refers to a preparation of exosomes that has been previously subjected to a lyophilization (i.e., freeze-drying) step. Thus, a "stable lyophilized exosome formulation" can refer to a dry exosome preparation or to a preparation of exosomes that has been subjected to lyophilization and subsequently been resuspended in an aqueous solution. The phrase, "suitable for administration to a human" means that the formulation has been prepared in a pharmaceutically acceptable composition under conditions to maintain sterility of the formulation.

The stable lyophilized exosome formulation can contain at least 10⁵, 5x10⁵, 10⁶, 5x10⁶, 10⁷, 5x10⁷, 10⁸, 5x10⁸, 10⁹, 5x10⁹, 10¹⁰, 5x10¹⁰, 10¹¹, 5x10¹¹, or 10¹², or 5x10¹² exosomes. In some embodiments, the stable lyophilized exosome formulation contains between 10⁵, 10⁶, 10⁷, 10⁸, 10⁹, 10¹⁰, or 10¹¹ to 10⁶, 10⁷, 10⁸, 10⁹, 10¹⁰, 10¹¹, or 10¹², etc. exosomes. Preferably, the stable lyophilized exosome formulation comprises at least 10⁶ or 10⁸ exosomes.

In some embodiments, the levels of miR-210 RNA and miR-146A in the stable lyophilized exosome formulation are maintained at a level of at least 10%, 20%, 30%, 40%, 50%, 60%, 70%, 80%, 90%, 95%, 99%, etc. of the formulation pre-lyophilization, preferably for at least 7, 14, 21, 23, 30, 60, 90, etc. days. Whether the levels of miR-210 RNA and miR-146A in the stable lyophilized exosome formulation are maintained at a level of at least 10%, 20%, etc. of the formulation pre-lyophilization for at least 7, 14, etc. days can be determined using the assays set forth in the Examples, and similar assays, i.e., by measuring the miRNAs pre- and post-lyophilization.

In some embodiments, the levels of miR-210 RNA and miR-146A are maintained at a level of at least 10%, 20%, etc. of the formulation pre-lyophilization for at least 7, 14, etc. days at 20°C, 10°C, 4°C, 0°C, -10°C, -20°C, -40°C, -80°C, etc. In some embodiments, the levels of miR-210 RNA and miR-146A are maintained at a level of at least 10%, 20%, etc. of the formulation pre-lyophilization for at least 7, 14, etc. days without refrigeration. Preferably, the levels of miR-210 RNA and miR-146A are maintained at a level of at least 70% of the formulation pre-lyophilization for at least 7 days at 4°C, -20°C, or -80°C. More preferably, the levels of miR-210 RNA and miR-146A are maintained at a level of at least 90% of the formulation pre-lyophilization for at least 7 days at 4°C, -20°C, or -80°C. Most preferably, the levels of miR-210 RNA and miR-146A are maintained at a level of at least 90% of the formulation pre-lyophilization for at least 7 days without refrigeration.

The invention encompasses a stable lyophilized exosome formulation comprising at least 10⁶ exosomes, wherein the wherein the lyophilized exosomes maintain at least 90% of the levels of miR-210 RNA and miR-146A of the exosome formulation pre-lyophilization.

Preferably, the stable lyophilized exosome formulation maintains at least 10%, 20%, 30%, 40%, 50%, 60%, 70%, 80%, 90%, 95%, 99%, etc. of the level of *in vitro* biological activity of the exosome formulation pre-lyophilization, preferably for at least 7, 14, 21, 23, 30, 60, 90, etc. days. The levels of pre- and post-lyophilization *in vitro* biological activity can be determined using the macrophage assay set forth in the Examples using the techniques of de Couto et al., J Clin Invest. 2015 Aug 3;125(8):3147-62, which is hereby incorporated by reference.

Preferably, the stable lyophilized exosome formulation maintains at least 10%, 20%, 30%, 40%, 50%, 60%, 70%, 80%, 90%, 95%, 99%, etc. of the level of *in vivo* biological activity of the exosome formulation pre-lyophilization, preferably for at least 7, 14, 21, 23, 30, 60, 90, etc. days. The levels of pre- and post-lyophilization *in vivo* biological activity can be determined using the rat AMI reperfusion model assay set forth in the Examples using the techniques of de Couto et al., J Clin Invest. 2015 Aug 3;125(8):3147-62, which is hereby incorporated by reference.

The invention encompasses a stable lyophilized exosome formulation comprising at least 10⁶ exosomes, wherein the lyophilized exosomes maintain at least 90% of the level of *in vitro* biological activity of the exosome preparation pre-lyophilization.

The invention encompasses a stable lyophilized exosome formulation comprising at least 10⁶ exosomes, wherein the lyophilized exosomes maintain at least 90% of the level of *in vivo* biological activity of the exosome preparation pre-lyophilization.

In some embodiments, the stable lyophilized exosome formulation comprises less than 1.0%, 0.1%, .01%%, 0.001%, 0.0001%, etc. polyethylene glycol (w/v). Preferably, stable lyophilized exosome formulation comprises less than .01% polyethylene glycol.

In some embodiments, the stable lyophilized exosome formulation the exosome formulation is generated from cardiosphere-derived cells (CDCs). In some embodiments, the stable lyophilized exosome formulation is generated from mesenchymal stem cells (MSCs), cardiac tissue derived stem cells, adipose tissue derived stem cells, neural tissue derived stem cells and other tissue derived stem cells.

Preferably, the stable lyophilized exosome formulation is generated by ultrafiltration. Most preferably, the stable lyophilized exosome formulation is generated by ultrafiltration from cardiosphere-derived cells (CDCs) by ultrafiltration.

### Harvesting exosomes

Exosomes can be harvested from cell cultures by routine techniques. For example, when the cells reach confluency, they can be washed three times in 25 ml PBS, 30 ml of IMDM is added (without FBS) and put back in an incubator at a specified concentration of oxygen. After a period of time, the IMDM media can be removed and placed in 50 ml conical tubes. The media can be centrifuged at 3000 xg for 15 minutes to eliminate cell debris. Media is separated into 10 ml fractions in 15 ml conical tubes and stored at -80°C.

Exosomes can be harvested after at least 1, 2, 3, 4, 5, 6, 7, 8, 9, 10, 11, 12, 13, 14, or 15 days in culture.

In some embodiments, exosomes are harvested every 2, 3, 4, or 5 days of culture.

### Purifying exosomes

Exosome preparations can be prepared by routine techniques in the art. In some embodiments, the preparation of exosomes includes centrifugation of the cells and/or media conditioned by the cells. In some embodiments, ultracentrifugation is used. In some embodiments, the preparation of exosomes from the population of cells is via size-exclusion filtration. In some embodiments, the preparation of exosomes from the population of cells includes use of discontinuous density gradients, immunoaffinity, ultrafiltration and/or high performance liquid chromatography (HPLC).

In some embodiments, differential ultracentrifugation is used, including using centrifugal force from at least 1000xg, 2000xg, 3000xg, 4000xg, 5000xg, 6000xg, 7000xg, 8000xg, or 9000xg, to 2000xg, 3000xg, 4000xg, 5000xg, 6000xg, 7000xg, 8000xg, 9000xg, 10,000xg, or larger to separate larger-sized particles from the exosomes derived from the cells.

In some embodiments, the preparation of exosomes from the population of cells includes use of filtration or ultrafiltration. In certain embodiments, a size exclusion membrane with different pore sizes is used. For example, a size exclusion membrane can include use of a filter with a pore size of at least 0.1, 0.5 µm, 1.0 µm, 2.5 µm, 5 µm, to 0.5 µm, 1.0 µm, 2.5 µm, 5 µm, or larger. In some embodiments, the pore size is about 0.2 µm. In some embodiments, filtration or ultrafiltration includes size exclusion ranging from 0.1 kDa, 0.5 kDa, 1 kDa, 2 kDa, 5 kDa, 10kDa, 25 kDa, 50 kDa, 100 kDa, or 250 kDa to 0.5 kDa, 1 kDa, 2 kDa, 5 kDa, 10kDa, 25 kDa, 50 kDa, 100 kDa, 250 kDa, 500 kDa, or more.

Preferably, isolated exosomes are filter sterilized with a 0.22 µm microbial exclusion filter. Preferably, exosomes are filtered using a 0.45 µm to remove cellular debris.

In various embodiments, such systems are used in combination with variable fluid flow systems. In other embodiments, the preparation of exosomes from the population of cells includes use of tangential flow filtration (TFF) systems are used purify and/or concentrate the exosome fractions. In other embodiments, the preparation of exosomes from the population of cells includes use of (HPLC) can also be used to purify exosomes to homogeneously sized particles. In various embodiments, density gradients as used, such as centrifugation in a sucrose density gradient or application of a discrete sugar cushion in preparation.

In other embodiments, the preparation of exosomes from the population of cells includes use of a precipitation reagent. For example, a precipitation reagent, such as EXOQUICK®, can be added to conditioned cell media to quickly and rapidly precipitate a population of exosomes. In some embodiments the preparation of exosomes from the population of cells includes use of volume-excluding polymers (e.g., polyethylene glycols (PEGs)). In another embodiment, the preparation of exosomes from the population of cells includes use of flow field-flow fractionation (FIFFF), an elution-based technique.

In some embodiments, PEG is used at a final concentration of 5%, 10%, 15%, or 20% to precipitate the exosomes. In some embodiments, the PEG has a molecular weight of about 4000, 6000, 8000, 10000, 12000, 15000, or 23000 Daltons. In some embodiments, the PEG has a molecular weight of about 4000-6000, 6000-8000, 8000-10000, 10000-12000, 12000-15000, or 15000-23000 Daltons.

In certain embodiments, the preparation of exosomes includes use of one or more capture agents to isolate one or more exosomes possessing specific biomarkers or containing particular biological molecules. In one embodiment, one or more capture agents include at least one antibody. For example, antibody immunoaffinity recognizing exosome-associated antigens is used to capture specific exosomes. In other embodiments, the at least one antibody are conjugated to a fixed surface, such as magnetic beads, chromatography matrices, plates or microfluidic devices, thereby allowing isolation of the specific exosome populations of interest.

In some embodiments, PEG is added to the exosome preparation after purification at a final concentration of 1-2%, 2-3%, 3-4%, 4-5% 5-6%, 6-7%, 7-8%, 8-9%, or 9-10%. In some embodiments, the PEG has a molecular weight of about 4000, 6000, 8000, 10000, 12000, 15000, or 23000 Daltons. In some embodiments, the PEG has a molecular weight of about 4000-6000, 6000-8000, 8000-10000, 10000-12000, 12000-15000, or 15000-23000 Daltons.

In some embodiments, an agent that causes aggregation of the exosomes is added to the exosome preparation prior to or after purification.

### Methods of making stable liquid exosome formulations

The invention encompasses methods of making stable liquid exosome formulations. In one embodiment, exosomes are subjected to ultrafiltration. Preferably, the ultrafiltration employs a 2, 3, 5, 10, 20, 40, 50, 80, or 100 kD filter.

In some embodiments, a microbial exclusion membrane is used to remove possible microbial contaminants (≤0.22 µm filter).

In some embodiments, the stable liquid exosome formulation is generated from cardiosphere-derived cells (CDCs). In some embodiments, the stable liquid exosome formulation is generated from mesenchymal stem cells (MSCs), cardiac tissue derived stem cells, adipose tissue derived stem cells, neural tissue derived stem cells and other tissue derived stem cells.

In some embodiments, the method comprises buffer exchange into physiologic buffered saline solution. Preferably, the method comprises diafiltration.

### Methods of making stable lyophilized exosome formulations

The invention encompasses methods of making stable liquid exosome formulations. In preferred embodiments, low temperature is maintained during the conditions of sublimation. In one embodiment, the protocol in the Examples is used for lyophilization.

In preferred embodiments, the exosomes are provided in a solution of IMDM or PLASMALYTE.

In one embodiment, the exosomes are frozen to -70°C prior to sublimation.

In one embodiment, the method comprises subjecting the exosomes to a vacuum at 200mT at -70°C. In various embodiments, the method comprises holding the exosomes at 100mT at -70°C for 80-480 min at a temperature between -40°C and -0°C, preferably at -40°C, -30°C, -10°C, or 0°C. Preferably, the vials containing the lyophilized exosomes are backfilled with Nitrogen.

In a preferred embodiment, the lyophilized exosomes are resuspended in an aqueous solution, preferably water.

### Methods of storing stable exosome formulations

In some embodiments, the invention encompasses method of storing stable exosome formulations. In some embodiments, the formulations are maintained at ambient room temperature. In some embodiments, the formulations are maintained at or below 20°C, 10°C, 4°C, 0°C, -10°C, -20°C, -40°C, -80°C, etc. In some embodiments, the formulations are maintained at ambient room temperature. In some embodiments, the formulations are maintained at a given temperature for at least 1 week, 1 month, 2 months, 3 months, 6 months, 1 year, etc. The invention encompasses exosome preparations that have been stored at or below 20°C, 10°C, 4°C, 0°C, -10°C, -20°C, -40°C, -80°C, etc. for at least 1 week, 1 month, 2 months, 3 months, 6 months, 1 year, etc.

### Analyzing exosomes

Exosomes preparations from cell cultured in less than 20% oxygen can be analyzed. The exosomes can be compared to exosomes prepared from similar cells cultured in 20% oxygen. Whether the protein or RNA content of the exosomes generated from cells cultured in less than 20% oxygen (e.g., in 2-8% oxygen) is higher than that of exosomes generated from cells cultured in 20% oxygen can be determined using the techniques set forth herein or by other similar techniques.

The number and size of the exosomes can be quantitated, for example using nanosight quantification.

The protein content of the exosomes can be analyzed using routine techniques to determining total protein levels or by using routine protein detection techniques (e.g., western blot) to determining the levels of specific proteins.

The RNA content of the exosomes can be analyzed using routine techniques to determining total RNA levels or by using routine nucleic acid detection techniques (e.g., PCR or probe hybridization) to determining the levels of specific RNAs. Preferred RNA are microRNAs, particularly miR-146A and miR-210 RNAs.

### EXAMPLES

### Example 1. Exosome Preparation

Immediately upon receipt, hearts were grossly dissected and cut into biopsy-sized pieces of about 25 mg each (500 µπι x 500 µπι x 500 µπι; though in some embodiments, other sizes are used), referred to as explants. Human hearts were cut using an automated tissue slicer (Zimmer® Dermatome) and automated tissue chopper (Mcllwain™ Tissue Chopper, Ted Pella, Inc.) as previously described (see e.g. United States Patent US20150216905 A1). Explants were then processed as previously described (see e.g., Smith et al. 2007 and United States Patent Application Nos. 11/666,685, filed April 21, 2007 and 13/412,051, filed March 5, 2012, the entireties of each of which are incorporated by reference herein).

In order to generate allogeneic CDCs, explants were plated on CELLBIND® CellSTACK® vessels (Corning Life Sciences). After 1-2 weeks, cellular outgrowth emerging from the explants became confluent. These explant derived cells (EDCs) were harvested using IX TrypLE™ (Invitrogen). EDCs were either cryopreserved as the master cell bank (MCB), and then cultured as cardiospheres (CSps), or placed immediately into CSp culture conditions. CSps were grown on UltraLow® CellSTACK® vessels (Corning Life Sciences).

Allogeneic CDCs were grown by seeding CSps on fibronectin-coated Nunc* TripleFlasks (Thermo Scientific), and passaging when confluent. CDCs at varying passage number were seeded on to fibronectin-coated CellBind cellstacks and allowed to become confluent for exosome production. Upon confluence, media was exchanged to serum-free conditions (e.g. Iscove's Modified Dulbecco's Media with HEPES and L-glutamine). Cells were allowed to condition media for 5 or 15 days.

### Example 2. Exosome Isolation

Exosomes were filtered using a 0.45 µm to remove cellular debris and then isolated by ultrafiltration based on size (2kda to 30 kda), polyethylene glycol precipitation or Exoquick (SBI, Mountain View, CA). In certain situations, isolated exosomes were filter sterilized with a 0.22 µm microbial exclusion filter. Exosomes were formulated using several diafiltrations to replace the buffer to an acceptable infusion solution (e.g. PLASMALYTE, RINGERS solutions).

### Example 3. Exosome Analysis

Exosomal protein was assessed using DC assay (Bio-Rad, Hercules, CA). Exosome particle size and concentration was assessed using Brownian motion and the Nanosight tracking analysis (Malvern Instruments Ltd, Malvern UK). RNA was isolated using miRNeasy micro kit (Qiagen, Valencia, CA) and quantified using either the Nanodrop, Qubit or AATI fragment analyzer (Advance Analytics, Ankeny, IA). Reverse transcription and qPCR reactions were conducted using TaqMan miR probes (ThermoFisher Scientific, Grand Island, NY).

### Example 4. Exosome Bioactivity

Bioactivity was assessed in vitro by the gene expression of bone marrow derived macrophages towards M1 or M2 phenotype as previously described (de Couto et al., J Clin Invest. 2015 Aug 3;125(8):3147-62). Bioactivity was assessed in vivo using a rat AMI reperfusion model as previously described (de Couto, et al 2015). All results are presented as mean + standard deviation. Statistical significance between two groups was determined using ANOVA to compare all groups followed by the Tukey HSD method to compare all pairs. Differences were considered significant for p<0.05.

### Example 5. Lyophilization protocol

Exosome formulations in media were lyophilized, while low temperatures were maintained during the conditions of sublimation. The lyophilization protocol was as follows:
Freeze @ -70°C as fast as possible and hold for -70°C for 60 min
Final Freeze @ -70°C for 90 min w/ vacuum start at 200mT
Primary Drying:
   Step 1 - ramp to -40°C in 0 min @ 100mT
   Step 2 - hold at -40°C for 90 min @ 100mT
   Step 3 - ramp to -30°C in 0 min @ 100mT
   Step 4 - hold at -30°C for 480 min @ 100mT
   Step 5 - ramp to -10°C in 0 min @ 100mT
   Step 6 - hold at -10°C for 240 min @ 100mT
   Step 7 - ramp to 0°C in 0 min @ 100mT
   Step 8 - hold at 0°C for 120 min @ 100mT
   Step 9 - ramp to +10°C in 0 min @ 100mT
   Step 10 - hold at +10°C for 90 min @ 100mT
Secondary:
   +20°C for 9,999min @ 100mT (note: 9,999 input to hold indefinitely)

The vials were backfilled with Nitrogen@ 700,000mT, stoppered under vacuum, and capped.

Embodiments of the invention are set out in the following numbered paragraphs:
1. A stable lyophilized exosome formulation suitable for administration to a human comprising at least 10⁶ exosomes, wherein the wherein the lyophilized exosomes maintain at least 90% of the levels of miR-210 RNA and miR-146A of the exosome formulation pre-lyophilization.
2. The stable lyophilized exosome formulation of paragraph 1, wherein the exosome formulation comprises at least 10⁸ exosomes.
3. The stable lyophilized exosome formulation of any of paragraphs 1-2, wherein the exosome formulation is generated from cardiosphere-derived cells (CDCs).
4. The stable lyophilized exosome formulation of paragraph 3, wherein the exosome formulation is generated from cardiosphere-derived cells (CDCs) by ultrafiltration and diafiltration.
5. A stable lyophilized exosome formulation suitable for administration to a human comprising at least 10⁶ exosomes, wherein the lyophilized exosomes maintain at least 90% of the level of *in vitro* biological activity of the exosome preparation pre-lyophilization.
6. The stable lyophilized exosome formulation of paragraph 5, wherein the exosome formulation comprises at least 10⁸ exosomes.
7. The stable lyophilized exosome formulation of any of paragraphs 5-6, wherein the exosome formulation is generated from cardiosphere-derived cells (CDCs).
8. The stable lyophilized exosome formulation of paragraph 7, wherein the exosome formulation is generated from cardiosphere-derived cells (CDCs) by ultrafiltration and diafiltration.
9. A stable lyophilized exosome formulation suitable for administration to a human comprising at least 10⁶ exosomes, wherein the lyophilized exosomes maintain at least 90% of the level of *in vivo* biological activity of the exosome preparation pre-lyophilization.
10. The stable lyophilized exosome formulation of paragraph 9, wherein the exosome formulation comprises at least 10⁸ exosomes.
11. The stable lyophilized exosome formulation of any of paragraphs 9-10, wherein the exosome formulation is generated from cardiosphere-derived cells (CDCs).
12. The stable lyophilized exosome formulation of paragraph 11, wherein the exosome formulation is generated from cardiosphere-derived cells (CDCs) by ultrafiltration and diafiltration.
13. A stable liquid cardiosphere-derived cell (CDC) exosome formulation suitable for administration to a human comprising at least 10⁶ CDC exosomes, wherein the exosome formulation maintains at least 75% of the levels of miR-210 RNA and miR-146A of the starting exosome formulation for 30 days at 4°C.
14. The stable liquid CDC exosome formulation of paragraph 13, wherein the exosome formulation comprises at least 10⁸ exosomes.
15. The stable liquid CDC exosome formulation of any of paragraphs 13-14, wherein the exosome formulation is generated from CDCs by ultrafiltration and diafiltration.

## Claims

1. A stable lyophilized exosome formulation suitable for administration to a human comprising at least 10⁶ exosomes, wherein the lyophilized exosomes maintain at least 90% of the levels of miR-210 RNA and miR-146A of the exosome formulation pre-lyophilization.

2. The stable lyophilized exosome formulation of claim 1, wherein the exosome formulation comprises at least 10⁸ exosomes.

3. The stable lyophilized exosome formulation of any of claims 1-2, wherein the exosome formulation is generated from cardiosphere-derived cells (CDCs).

4. The stable lyophilized exosome formulation of claim 3, wherein the exosome formulation is generated from cardiosphere-derived cells (CDCs) by ultrafiltration and diafiltration.

5. A stable lyophilized exosome formulation suitable for administration to a human comprising at least 10⁶ exosomes, wherein the lyophilized exosomes maintain at least 90% of the level of *in vitro* biological activity of the exosome preparation pre-lyophilization.

6. The stable lyophilized exosome formulation of claim 5, wherein the exosome formulation comprises at least 10⁸ exosomes.

7. The stable lyophilized exosome formulation of any of claims 5-6, wherein the exosome formulation is generated from cardiosphere-derived cells (CDCs).

8. The stable lyophilized exosome formulation of claim 7, wherein the exosome formulation is generated from cardiosphere-derived cells (CDCs) by ultrafiltration and diafiltration.

9. A stable lyophilized exosome formulation suitable for administration to a human comprising at least 10⁶ exosomes, wherein the lyophilized exosomes maintain at least 90% of the level of *in vivo* biological activity of the exosome preparation pre-lyophilization.

10. The stable lyophilized exosome formulation of claim 9, wherein the exosome formulation comprises at least 10⁸ exosomes.

11. The stable lyophilized exosome formulation of any of claims 9-10, wherein the exosome formulation is generated from cardiosphere-derived cells (CDCs).

12. The stable lyophilized exosome formulation of claim 11, wherein the exosome formulation is generated from cardiosphere-derived cells (CDCs) by ultrafiltration and diafiltration.

13. A stable liquid cardiosphere-derived cell (CDC) exosome formulation suitable for administration to a human comprising at least 10⁶ CDC exosomes, wherein the exosome formulation maintains at least 75% of the levels of miR-210 RNA and miR-146A of the starting exosome formulation for 30 days at 4°C.

14. The stable liquid CDC exosome formulation of claim 13, wherein the exosome formulation comprises at least 10⁸ exosomes.

15. The stable liquid CDC exosome formulation of any of claims 13-14, wherein the exosome formulation is generated from CDCs by ultrafiltration and diafiltration.
